(19) Europäisches Patentamt

European Patent Office

Office européen des brevets

(11) EP 0 218 479 B2

(12) NEW EUROPEAN PATENT SPECIFICATION

(45) Date of publication and mention
of the opposition decision:
**05.08.1998 Bulletin 1998/32**

(45) Mention of the grant of the patent:
**22.06.1994 Bulletin 1994/25**

(21) Application number: **86307646.9**

(22) Date of filing: **03.10.1986**

(51) Int Cl.[6]: **C12Q 1/56**, C12N 9/64,
A61K 38/56

(54) **Process for the production of a plant protein reagent, binding to tPA**

Verfahren zur Herstellung eines Pflanzenproteinreagens, das an tPA bindet

Procédé pour la production d'un réactif protéique végétal liant du tPA

(84) Designated Contracting States:
**AT CH DE FR GB LI SE**

(30) Priority: **04.10.1985 ZA 857662**

(43) Date of publication of application:
**15.04.1987 Bulletin 1987/16**

(73) Proprietor: **SOUTH AFRICAN INVENTIONS
DEVELOPMENT CORPORATION
Pretoria Transvaal (ZA)**

(72) Inventors:
• **Dowdle, Eugene Bernard Davey
Bishopscourt Cape Town 7700 (ZA)**
• **Joubert, Francois Johannes
Nat. Chem. Res. Lab.
Scientia Meiringnaude Road Pretoria (ZA)**

(74) Representative: **Woodman, Derek et al
Frank B. Dehn & Co.,
European Patent Attorneys,
179 Queen Victoria Street
London EC4V 4EL (GB)**

(56) References cited:
• HOPPE-SEYLER'S Z. PHYSIOL. CHEM., vol. 362, May 1981, pages 531-538, Walter de Gruyter & Co., Berlin, DE; F.J. JOUBERT et al.: "Purification and some properties of two proteinase inhibitors (DE-1 and DE-3) from Erythrina latissima (broad-leaved Erythrina) seed"
• JOURNAL OF BIOLOGICAL CHEMISTRY, vol. 260, no. 24, 25th October 1985, pages 12948-12953, The American Society of Biological Chemists Inc.; F.J. JOUBERT et al.: "The complete amino acid sequence of trypsin inhibitor DE-3 from Erythrina latissima seeds"
• PHYTOCHEMISTRY, vol. 21, no. 6, 1982, pages 40-44; F.J. JOUBERT: "Proteinase inhibitors from Erythrina lysistemon seed"
• JOURNAL OF BIOCHEMISTRY, vol. 94, no. 3, 1983, pages 849-863; M. YAMAMOTO et al.: "Amino acid sequences of two trypsin inhibitors from winged bean seeds (Psophocarpus tetragonolobus (L)DC.)"
• G Biol. Chem. 259 (1984) 11635-8.

Remarks:
The file contains technical information submitted after the application was filed and not included in this specification

## Description

The present invention relates to processes for the production of a protein reagent having the ability to bind tissue plasminogen activator in its two chain form (tPA) and/or its single chain form (hereinafter referred to as pro-tPA) reversibly from aqueous media containing it, and of immobilised forms of said reagent.

The reagent has the unique property of selectivity for tPA and pro-tPA to the exclusion of other plasminogen activators, even urokinase which in some tissues occurs side by side with tPA.

Recombinant DNA technology has recently made available mutagenic variants of tPA and pro-tPA which differ from the natural proteins by replacement, removal or addition of one or more amino acid residues. The terms "tPA" and "pro-tPA" as used herein include such variants provided they retain the site or sites having an affinity for the reagent of the invention.

Plasminogen activators are enzymes which by their action upon plasminogen (a precursor of plasmin) result in the formation of plasin. Plasmin in turn acts upon fibrin (or blood clots) to liquefy or dissolve the fibrin. Plasmin also causes lysis of fibrinogen which is a precursor of fibrin.

The aforegoing effects play an important role in the natural fibrinolytic systems. They are also put to use in the therapeutic administration of plasminogen activators for the management, treatment or prophylaxis of thrombotic disease or other conditions where it is desirable to produce local fibrinolytic or proteolytic activity via the mechanism of plasminogen activation.

They may also find use in reagents for diagnostic, pathological or scientific tests involving fibrinolysis in vitro.

Two types of activators of human plasminogen are extensively used. The first of these is the bacterial enzyme, streptokinase, which functions by a non-proteolytic mechanism. The second is the protease urokinase, which is obtained from urine or cultured kidney cells, and by recombinant DNA procedures. These two compounds have the disadvantage that their action is not localised in that it is not confined to the fibrin in the blood clot. They act upon plasminogen generally in the circulation and, in consequence, produce widespread plasmin generation with extensive lysis of fibrinogen, the precursor of fibrin. This may in turn lead to a widespread systemic bleeding as a result of ineffective coagulation of the blood.

The tendency for tPA to bind to fibrin, its greatly enhanced fibrinolytic action in the presence of fibrin and when compared to urokinase and streptokinase and its relatively inefficient function as a plasminogen activator in the absence of fibrin combine to make tPA the therapeutic drug of choice for human thrombolytic therapy. Interactions between fibrin and tPA to a considerable extent localise the plasmin generation to the site of the clot and mitigate or avoid the consequences of promiscuous plasminogen activation as observed when urokinase or streptokinase is used. Furthermore, tPA is a protein which is immunochemically compatible with man, whereas streptokinase is not, being a foreign protein.

According to P. Wallen et al (Prog. Chem. Fibrinolysis Thrombolysis 5. 16-23 (1981) tPA occurs in two different forms, one being a single-chain form which is converted by plasmin or trypsin into two chains linked by disulphide bridges.

Human tPA is present in tissue extracts, vessel perfusates and mammalian cell cultures. More recently, attention has been given to secure the expression of tPA by recombinant DNA technology in certain mammalian host cells, yeasts and bacteria. To produce tPA efficiently on a commercial scale, a method is required to easily extract the tPA enzyme occuring in small quantities in a complex mixture of interfering proteins, cell debris and the like which are present in the cell culture medium. Moreover, such a method must permit high recovery of tPA from the harvest fluid and produce a product of high purity if the enzyme is to be administered intravenously to human patients. Also, a method must permit a high rate of production if it is to be used on a commercial scale.

Japanese Patent Application No. 5148/83 describes an affinity reagent and process for selectively and reversibly adsorbing tPA and/or pro-tPA from aqueous media, capable of achieving a high degree purification and high yields of tPA, often substantially in a single step. This affinity reagent is based on an immobilised chromatographic protein fraction numbered DE-3 exhibiting tPA enzyme -inhibiting properties, and may be obtained from seeds of Erythrina species such as E. latissima; it is distinguished from other enzyme inhibitors occuring in such species by the following combination of characteristics:

(a) it is an inhibitor of the Kunitz type;
(b) it has no effect on urokinase;
(c) it inhibits tPA and/or pro-tPA; and
(d) it inhibits trypsin and plasmin.

It also has no effect on thrombin.

The seeds of Erythrina latissima (broad-leafed Erythrina) and other Erythrina species contain several proteinase inhibitors (Joubert et al., Hoppe-Seyler's Z. Physiol. Chem., 362 531 538 (1981)). Two chromatography fractions being the first and third eluates from a DEAE sepharose column and referred to in that publication as DE-1 and DE-3 respectively showed trypsin inhibition activity. In a subsequent publication (Francois J. Joubert, Phytochem., 21, (6), 1213-1217, (1982)) four separate protein fractions (arbitrarily numbered DE-1, DE-2, DE-3 and DE-4) were extracted chromatographically from Erythrina lysistemon seed. Again all four fractions displayed trypsin inhibition activity.

It was subsequently found that the DE-3 fraction from E.latissima possessed the valuable property of inhibiting tPA and pro-tPA in addition to the inhibition of trypsin reported by Joubert et al. This discovery was disclosed in the above Japanese Patent Application. The present inventors have since discovered that seeds of other Erythrina species also contain fractions similar to the above DE-3 fractions which are capable of selectively inhibiting tPA and pro-tPA. The DE-3 protein fraction and similar fractions inhibiting tPA and pro-tPA (not necessarily eluted from DEAE-sepharose in the same order) have been found to occur in the seeds of all of the numerous Erythrina species tested to date and are therefore considered to be common to substantially all Erythrina species.

The DE-3 fraction and similar fractions from seeds of Erythrina species are the only known proteinase inhibitors not extracted from body fluids having the property of inhibiting tPA.

The above contribution to the art, although of great value was based on an incomplete understanding and characterisation of the substances involved. In particular, it was not realised at the time that the above tPA-inhibiting fractions then claimed were not pure and indeed had only a fraction of the specific tPA-inhibiting capacity of the pure substance responsible for the tPA-inhibiting properties. The present inventors have now succeeded in a much more complete purification of these tPA-inhibiting fractions and have identified and characterised the active tPA inhibitor protein.

As a surprising result of such purification, it is found that the original DE-3 product from E.latissima and the corresponding tPA inhibiting fractions from other Erythrina species contained contaminating proteolytic enzymes which are capable of degrading tPA and /or pro-tPA. Thus, when these are removed, the resulting purified inhibitor is greatly increased in efficiency, in that virtually no proteolytic degradation of the desired tPA and/or pro-tPA is observed and the therapeutic quality of the tPA and/or pro-tPA is thus considerably enhanced. This is surprising, since the previous publications concerning DE-3 indicated this to be substantially homogeneous.

According to the present invention, therefore, we provide a process for the production of a protein reagent having the ability to bind to tPA and/or pro-tPA reversibly comprising a Kunitz-type proteinease inhibitor obtainable from seeds of an Erythrina species and having a molecular weight in the range 17-23,000 daltons, being non-cytotoxic, having the ability to inhibit tPA, pro-tPA and trypsin, but not urokinase or thrombin, having two disulphide bridges, having Val as the N-terminal amino acid residue and being free from proteases, wherein an aqueous solution containing said reagent together with contaminants is subjected to one or more fractionation steps including (a) chromatography using a hydrophobic gel; (b) size-exclusion chromatography; and (c) ion-exchange chromatography; with selection of those fractions identified as containing said reagent and with exclusion of any fractions containing proteases.

The above Kunitz-type inhibitor also inhibits plasmin and elutes from a DEAE-sepharose column using a linear sodium chloride gradient (0-0.2M over 1 litre) in 0.05M Tris/HCl buffer at pH 8, at 20°c and a flow rate of 20ml/hour, at a retention volume in the range 600-690mls.

As indicated above, the protein reagent can be obtained from seeds of Erythrina species, for example E. caffra, E. latissima, E. acanthrocarpa, E. corallodendron, E. lysistemon. E. humeana, E. seyheri and E. decora.

The Kunitz-type inhibitor which is the active agent produced by the process of the invention may have the following amino acid sequence:

3

EP 0 218 479 B2

```
                                                 10
H₂N-Val-Leu-Leu-Asp-Gly-Asn-Gly-Glu-Val-Val-

                                                 20
Gln-Asn-Gly-Gly-Thr-Tyr-Tyr-Leu-Leu-Pro-

                                                 30
Gln-Val-Trp-Ala-Gln-Gly-Gly-Gly-Val-Gln-

                                                 40
Leu-Ala-Lys-Thr-Gly-Glu-Glu-Thr-Cys-Pro-

                                                 50
Leu-Thr-Val-Val-Gln-Ser-Pro-Asn-Glu-Leu-

                                                 60
Ser-Asp-Gly-Lys-Pro-Ile-Arg-Ile-Glu-Ser-

                                                 70
Arg-Leu-Arg*-Ser-Ala-Phe-Ile-Pro-Asp-Asp-

                                                 80
Asp-Lys-Val-Arg-Ile-Gly-Phe-Ala-Tyr-Ala-

                                                 90
Pro-Lys-Cys-Ala-Pro-Ser-Pro-Trp-Trp-Thr-

                                                 100
Val-Val-Glu-Asp-Glu-Gln-Glu-Gly-Leu-Ser

                                                 110
Val-Lys-Leu-Ser-Glu-Asp-Glu-Ser-Thr-Gln-

                                                 120
Phe-Asp-Tyr-Pro-Phe-Lys-Phe-Glu-Gln-Val-

                                                 130
Ser-Asp-Gln-Leu-His-Ser-Tyr-Lys-Leu-Leu

                                                 140
Tyr-Cys-Glu-Gly-Lys-His-Glu-Lys-Cys-Ala

                                                 150
Ser-Ile-Gly-Ile-Asn-Arg-Asp-Gln-Lys-Gly-

                                                 160
Tyr-Arg-Arg-Leu-Val-Val-Thr-Glu-Asp-Tyr-

                                                 170
Pro-Leu-Thr-Val-Val-Leu-Lys-Lys-Asp-Glu-Ser-Ser-OH
```

and comprises two disulphide bridges in the following positions:

Cys[39] to Cys[83] and Cys[132] to Cys[139], or has a functionally analogous amino acid sequence having the property of inhibiting tPA and/or pro-tPA, including derivatives and fragments of the aforesaid polypeptide in which the property of inhibiting tPA or pro-tPA is preserved.

4

The presence of the two disulphide bridges (or of at least either of these) is important for the inhibiting effect towards tPA and pro-tPA. Breaking of the chain with trypsin whilst leaving the disulphide bridges intact did not result in loss of activity. However, the reduction of the disulphide bridges resulted in loss of activity. This leads to the conclusion that the geometry or three-dimensional conformation is of some signficance in order to determine whether or not a molecule having substantially the aforesaid amino acid sequence inhibits tPA and pro-tPA.

There are also strong indications that it is important for the N-terminal amino acid to be valine since this is not normally present in Kunitz-type protease inhibitors but is present in all the tPA inhibitors from Erythrina species which have been investigated.

However, it appears that some modification or degradation of the Kunitz-type inhibitor may be effected without loss of tPA binding activity. Thus, as indicated above, some limited proteolysis of the molecule, in particular cleavage at the trypsin binding site, does not destroy the tPA binding activity provided the disulphide bonds are retained. Further the trypsin binding site may be blocked to produce a biosorbant of even greater selectivity. Thus, for example, the inhibitor may be reacted with an arginine blocking agent such as 1,2-cyclohexane-dione to block the trypsin binding site. If required, such blocking may be removed by treatment with hydroxylamine.

It can be assumed that, for the desired tPA-inhibitory activity to be present, the homology with the above amino acid sequence exceeds 85%, preferably 90%, at least in the region which includes positions 39 to 139 of the amino acid sequence, in particular in the region between about positions 50 to 80.

The amino acid compositions of the Kunitz-type inhibitors having tPA inhibiting activity, as obtained from the different Erythrina species examined, are closely similar but show some variation as shown hereinafter. In general, the amino acid compositon falls within the following limiting values:-

Asp (15-18), Thr (7-9), Ser (12-14), Glu (21-26), Pro (10-14), Gly (14-15), Ala (6-8), Cys (4), Val (15-16), Met (0-1), Ile (6-7), Leu (16-17), Tyr (7-8), Phe (4-6), Lys (10-13), His (1-3), Arg (5-8) and Trp (2-3). The total number of amino acid residues in each case is 172. As indicated above, two disulphide bonds are present in each case and the N-terminal amino acid is always valine.

The Kunitz-type tPA inhibitor from E. latissima has the following amino acid sequence:

```
                                          10
H₂N-Val-Leu-Leu-Asp-Gly-Asn-Gly-Glu-Val-Val-

                                          20
Gln-Asn-Gly-Gly-Thr-Tyr-Tyr-Leu-Leu-Pro-

                                          30
Gln-Val-Trp-Ala-Gln-Gly-Gly-Gly-Val-Gln-

                                          40
Leu-Ala-Lys-Thr-Gly-Glu-Glu-Thr-Cys-Pro-

                                          50
Leu-Thr-Val-Val-Gln-Ser-Pro-Asn-Glu-Leu-

                                          60
Ser-Asp-Gly-Lys-Pro-Ile-Arg-Ile-Glu-Ser-

                                          70
Arg-Leu-Arg*-Ser-Thr-Phe-Ile-Pro-Asp-Asp-

                                          80
Asp-Glu-Val-Arg-Ile-Gly-Phe-Ala-Tyr-Ala-

                                          90
Pro-Lys-Cys-Ala-Pro-Ser-Pro-Trp-Trp-Thr-

                                          100
Val-Val-Glu-Asp-Glu-Gln-Glu-Gly-Leu-Ser

                                          110
Val-Lys-Leu-Ser-Glu-Asp-Glu-Ser-Thr-Gln-

                                          120
Phe-Asp-Tyr-Pro-Phe-Lys-Phe-Glu-Gln-Val-

                                          130
Ser-Asp-Lys-Leu-His-Ser-Tyr-Lys-Leu-Leu

                                          140
Tyr-Cys-Glu-Gly-Lys-His-Glu-Lys-Cys-Ala

                                          150
Ser-Ile-Gly-Ile-Asn-Arg-Asp-Gln-Lys-Gly-

                                          160
Tyr-Arg-Arg-Leu-Val-Val-Thr-Glu-Asp-Asn-

                                          170
Pro-Leu-Thr-Val-Val-Leu-Lys-Lys-Asp-Glu-Ser-Ser-OH
```

More particularly, the protein reagent is such that in its concentrated form, prior to any dilution with a solvent, diluent or extender it has a specific activity of at least 60 000 IU/mg (as herein defined), preferably upward of 70000, IU/mg. The tPA inhibitor preferably comprises at least 80% by weight, more preferably at least 90% by weight, of the protein reagent.

Furthermore, the increased purity of the active protein results in surprisingly higher specific activity. Thus, for example, the DE-3 chromatography eluate fractions described previously had specific tPA inhibition activities below 40 000 IU/mg and often well below that, whereas purified fractions have now been obtained according to the process of the invention which have specific activities of 90,000 IU/mg or greater. When the protein is immobilised on a matrix to provide an affinity reagent, it is found that the resulting much higher loading of active protein on to the matrix enables the fluids containing tPA and/or pro-tPA to be processed much more rapidly, thus reducing the spontaneous degradation normally observed on prolonged handling of sensitive proteins. This also results in savings in matrix material and processing time.

We have observed that the impurities present in the DE-3 fraction and similar tPA inhibiting fractions as described previously include other Erythrina protease inhibitors. These other protease inhibitors as indicated hereinafter have molecular weights closely similar to those of the respective tPA inhbitors. When these other protease inhibitors are present in the immobilised form of the reagent, they are capable of adsorbing other proteases in addition to tPA and pro-tPA so that on desorption, the product will be significantly less pure. Such other proteases are commonly present in both the tissue fluids and the cell culture media from which tPA and pro-tPA are normally isolated.

The reagent produced by the process of the invention may advantageously be used in the immobilised form. Such an immobilised reagent is highly stable and maintains its specificity for tPA even after extensive repeated use, possibly due to the absence of proteases in the immobilised reagent. Thus it lends itself to the extraction and purification of tPA from complex protein mixtures on an industrial scale requiring repeated sorption and desorption to tPA without degradation or denaturation of the affinity reagent and without the immobilised reagent losing its specificity to tPA. The column can be cleaned and sterilised without adversely affecting the stability of the immobilised reagent. The rate of binding of tPA to the immobilised reagent is high. Thus the flow rate through a column of the immobilised reagent is substantially higher than say in the case where size exclusion chromatography is used to achieve a separation of tPA from the many other interfering proteins present in the cell harvest fluid.

Methods of immobilising the protein reagent according to the invention included methods of covalently bonding the reagent to a carrier. For example. the reagent of the invention may be coupled to cyanogen bromide activated agarose in a manner known as such to persons skilled in the art. However, other solid carriers can be used and other coupling agents depending on the chemical propeties of the surface to which the protein reagent is to be coupled. Examples are:

(a) carbodiimide coupling which couples free amino groups to free carboxy groups;

(b) glutaraldehyde coupling which couples $NH_2$ groups to agarose previously converted into the aminoalkyl form;

(c) periodate activation of agarose to generate aldehyde functions which are subsequently reacted at pH 4 to 6 with amines of the inhibitor to form Schiff bases which are then reduced with sodium borohydride or sodium cyano borohydride

(d) agarose may be converted into the hydraziosuccinyl derivative; to this carboxylic ligands may be attached with EDC, or amines by diazotisation;

(e) cellulose fibres or beads may be converted into a hydrazide derivative and used according to the method of Parikh et al (Methods In Enzymology Vol. 34. pp 77 to 102, ed. Jakobi and Wilcheck, Academic press NY).

(f) polyacrylamide beads may be coupled by direct glutaraldehyde procedure or by diazotisation of the p-aminobenzamido ethyl derivatives or of the hydrazido derivative;

(g) finally, reference is made to glass beads (or other solid bodies) which can be coaled with a protein, e.g. gelatin which may be cross linked and coupled to the inhibitor by methods analogous to any of the coupling methods described in German patent application P3224387.7, Japanese patent application 116086/1982. United Kingdom patent 2103791 and US patent 4478946.

The aforegoing methods are known as such to persons skilled in the art and/or are described in the cited literature.

The processes of using the immobilised reagent disclosed in the aforementioned Japanese Patent Application No. 5148/83 can be applied identically to the affinity reagents as disclosed in the present specification. This applies to the specific examples as well.

Human tissue plasminogen activator is present in blood, tissue extracts, vessel perfusates and cell cultures but in extremely low concentrations. An alternative source of tPA is listed in EPO application No. 81200643-5 which describes a melanoma cell culture capable of producing tPA in higher concentrates than generally found in the aforegoing sources.

It is found that pro-tPA is converted to tPA by the action of proteases. Consequently, a further advantage of the removal of proteases from the reagent of the invention could be an improvement in the ratio of pro-tPA to tPA in the final product. It has been suggested that pro-tPA may be preferred to tPA for administration because it is less active in the absence of fibrin, but is strongly absorbed selectively by fibrin clots; once absorbed it is rapidly converted to tPA and initiates lysis of the clot.

More recently, increasing attention has been given to the application of recombinant DNA techniques for the expression of tPA in relatively high concentration, mainly in mammalian cell lines, fibroblasts, yeasts and bacteria. By such recombinant DNA technology, the human gene encoding for tPA is transvected into the host cell which is thereby transformed to synthesise the tPA enzyme. By the method of gene amplification an increase in the concentration of tPA is obtained. These procedures are described in S.A. Patent Application 83/3174 and EPO Publication Nos 117 059 and 117 060. As indicated above, such techniques are capable of providing mutagenic variants of tPA and pro-tPA.

The protein reagent produced by the process of the invention does not inhibit or react with urokinase and can therefore be used advantageously in immobilised form to isolate tPA and/or pro-tPA from aqueous solutions containing them in conjunction with urokinase, for example various body tissue fluids. Furthermore, it may be possible to isolate a mixture of tPA, pro-tPA and urokinase from such fluids using a less selective biosorbant and then to use the reagent produced by the process of the invention to remove the tPA and pro-tPA, thereby yielding purified urokinase.

The present teachings are based on a substantially improved process for the purification of the active protein reagent from its natural sources, in particular seeds of Erythrina species, which provide the inhibitor substantially free from protease activity.

Thus, according to a further aspect of the present invention, we provide a process for the production of a protein reagent of the present invention having the ability to bind tPA and/or pro-tPA reversibly wherein an aqueous solution containing said reagent together with contaminants, e.g. an aqueous extract of seeds of an Erythrina species, is subjected to one or more fractionation steps, including chromatography using a hydrophobic get with selection of those fractions identified as containing the desired reagent and with exclusion of any fractions containing proteases.

A process according to the present invention may be similar in some respects to the method described by Joubert (loc. cit.).

However, it has been found surprisingly that substantially improved purificatious are achieved if the fractionation steps include hydrophobic chromatography using a hydrophobic get, most especially phenyl agarose. Use of phenyl sepharose (CL 4B) or a technical equivalent is particularly preferred. The desired protein is eluted at pH 6.0-8.0, preferably about pH 7.0.

The above-described exclusion chomatography step is preferably combined with ion exchange chromatography on DEAE cellulose, e.g. DEAE-cellulose DE-52 or a technical equivalent, and/or size-exclusion chromatography, for example on a cross-linked dextran adsorbant such as Sephadex G-50 or a technical equivalent, following an initial fraction by ammonium sulphate precipitation.

Thus, for example, in a preferred process according to the invention exclusion chromatagraphy using phenyl sepharose is preceded by chromatography on DEAE-cellulose, preferably at pH 7-9, most preferably about pH 8.0 and is followed by chromatography on Sephadex G-50 or a technical equivalent, preferably at pH 7-9, most preferably at pH 8.2-8.3.

It appears from our experiments that it is chromatography on a hydrophobic get such as phenyl sepharose, which is responsible for the removal of proteases from the Kunitz-type inhibitor.

The following non-limiting Examples are intended to illustrate the present invention.

Example 1

Preparation of tPA inhibitor from seeds of an Erythrina species

Ground defatted seeds of an Erythrina species, e.g. E. Latissima, (10 kg) are extracted with 100 l of 0.5 M sodium chloride solution overnight at 10°C. The suspension is then macerated for 5 min in a Waring blender. The extract is clarified, brought to 60% saturation with ammonium sulphate and the precipitate recovered by centrifugation. The precipitate is redissolved in 0.05 M sodium chloride, dialysed against distilled water and lyophilised.

The crude extract is further fractionated on DEAE-cellulose (DE 52) using a linear sodium chloride gradient (0-0.2 M) in 0.05 M Tris/HCl at pH 8. The 0.2 M NaCl fraction is collected and loaded on to a phenyl sepharose column (Cl 4B). The column is eluted with 10% w/v ammonium sulphate in 0.01 M Na P, pH 7.0 and the tPA inhibitor peak collected.

The partially purified tPA inhibitor is then loaded on to a Sephadex G-50 column and eluted with 0.2 M ammonium bicarbonate, pH 8.2.

Using the above method, over 90% pure tPA inhibitor has been obtained, free of detectable amounts of proteases.

Example 2

Assay for tPA-inhibitor activity

The inhibitory activity of a tPA inhibitor derived from seeds of an Erythrina species can be expressed in terms of inhibitor units which are defined experimentally as follows:

(i) A sample of pure tPA is assayed fluorometrically by the method of Zimmerman et al (Proc. Natl. Acad. Sci. USA (1978), 75, 750) using the fluorogenic substrate n-alpha-benzyloxcarbonyl-glycyl-glycyl-L-arginine-7-amido-4-methylcoumarin (Cbz-Gly-Gly-Arg-AMC; Bachem, Switzerland) and adjusted to contain 50 fluorometric units of enzyme/ml of 0.1 M Tris-HCl containing 0.02% Triton X-100.

(ii) The tPA inhibitor sample is dissolved in 0.1 M Tris-HCl pH 8.1 at a convenient concentration (usually 0.1-2mg/ml, depending upon the expected specific activity of the inhibitor).

(iii) Serial two-fold dilutions of the inhibitor solution are prepared in 0.1 M Tris-HCl, pH 8.1 to a final dilution of 1/2048.

(iv) Each inhibitor dilution (10 µl volume) is mixed with 90 µl of the tPA solution and the tubes are incubated for 30 minutes at room temperature.

(v) Each tube is then assayed for residual activity and the logit transform of fractional residual activity is plotted as a function of the logarithm of the inhibitor dilution. The least squares regression line is fitted to these points and used to obtain the coordinates for "50%" inhibition" by interpolation.

(vi) One unit of inhibitory activity is defined as that amount that will inhibit 1 fluorometric unit of tPA by 50%.

The standard assay employed is performed by adding enzyme to initiate the reaction in the following mixture.

|  | ml |
| --- | --- |
| Tris-HCl 0.1 M, pH 8.1 | 0.43 |
| Substrate 25 mM in DMSO | 0.01 |
| DMSO | 0.01 |
| Enzyme solution | 0.05 |
| TOTAL | 0.05 ml |

The mixture is vortexed briefly and placed in the sample chamber of a spectrofluorophotometer equipped with a recorder. Release of AMC is monitored at an activating wavelength of 383 nm and an emitting wavelength of 455 nm.

The instrument is standardised with pure AMC so that a full scale recorder deflection is given by the above assay mixture in which 10 µl of $2 \times 10^{-5}$ M AMC in DMSO replaces the 10 µl of DMSO (i.e. a full scale recorder deflection is given by 200 pmoles of AMC or by 0.5 ml of a $4 \times 10^{-7}$ M solution of AMC in the assay mixture).

One fluorometric unit of the enzyme activity is defined as that amount that will catalyse the release of 10 p moles of AMC/min at 20°C.

The following is a specific example of such determination:

A partly purified fraction containing tPA inhibitor freeze-dried at 5mg/vial and reconstituted at 2.5 mg/ml.

Doubling dilutions performed in 0.1 M Tris HCl pH 8.1.

Standard tPA solution 47.3 u/ml

10 µl inhibitor dilution + 90µl tPA; 30 min at room temperature; assay enzyme activity in mixture.

| Results | | | | |
| --- | --- | --- | --- | --- |
| Sample | Inhibitor dilution | Enzyme activity (FU/ml) | Residual activity (R) (%) $(\text{Logit} = \log (\frac{R}{100\text{-}R}))$ | |
| 1 | No | 42.6 | 100 | - |
| 2 | 1/16 | 2.22 | 5.2 | - 1.261 |
| 3 | 1/32 | 3.98 | 9.34 | - 0.857 |
| 4 | 1/64 | 6.69 | 15.70 | - 0.730 |
| 5 | 1/128 | 11.08 | 26.00 | - 0.454 |
| 6 | 1/256 | 16.64 | 39.05 | - 0.193 |
| 7 | 1/512 | 23.00 | 54.00 | 0.397 |
| 9 | 1/2048 | 34.08 | 80.00 | 0.602 |

Regression equation:-

$$\text{Logit } R = 0.885 \log (I/\text{dil}) - - 2.3232$$

where R = residual activity and logit

$$R = \log \frac{R}{(100\text{-}R)}$$

50% inhibitory dilution = 1/423
10 µl of a 1/423 dilution of 2.5 mg/ml inhibitor solution inhibited 50% of 4.26 units.
Specific activity is therefore 4.26 units x 1/0.01 ml x 423 x 1/2.5 mg = 72 100 units /mg.

Example 3

Seeds of the following species of Erythrina were processed as in Example 1 up to and including the stage of DEAE-cellulose chromatography. The preconcentrates of tPA inhibitor so obtained had the following activities as determined by the method of Example 2.

| SPECIES | IU/mg |
|---|---|
| E. lysistemon | 27 247 |
| E. decora. | 20 833 |
| E. seyheri. | 15 797 |
| E. humeana. | 20 533 |
| E. abussinia. | 5 050 |

By following the procedures as described in Example 1, these materials can be upgraded. For example, the E. lysistemon preconcentrate was upgraded from 27 246 IU/mg to 94 339 IU/mg, whilst the E. seyheri preconcentrate was upgraded from 15 797 IU/mg to 71 428 IU/mg.

Example 4

Alternative procedure for preparation of tPA inhibitor from seeds of an Erythrina species

1. Grind seeds of an Erythrina species to a fine powder in a coffee mill.
2. Take 200g powdered seed and extract with 2 litres of 0.5M NaCl. Stir overnight at 4°C.
3. Filter through glass wool and clarify solution by centrifugation for 1 hour at 10,000 rpm. Keep supernatant and discard residue.
4. Extract seed slurry a second time with 2 litres of 0.5M NaCl, filter, centrifuge and combine supernatant with that from first extract.
5. Add ammonium sulphate to the combined extracts to give 60% saturation (390 g/litre). Stir gently overnight at 4°C.
5. Centrifuge solution at 5000 rpm for 30 min, discard supernatant and re-dissolve the precipitate in 0.5M NaCl (approx. 200 ml).
7. Dialyse overnight against running tap water at 4°C.
8. Centrifuge at 5000 rpm for 30 min.
9. Freeze-dry supernatant liquid to obtain crude inhibitor (yield approx 4g/200g seed powder).
10. Dissolve the crude inhibitor to a concentration of 50 mg per ml in 0.05M Tris/HCl at pH 8.0.
11. Load on to a column of DEAE cellulose (DE52).
12. Wash with 0.1M NaCl in Tris/HCl at pH 8.0.
13. Elute with 0.22M NaCl in 0.05M Tris/HCL at pH 8.0.
14. Dialyse and freeze dry.
15. Dissolve to a concentration of 50 mg protein/ml in 0.01M $NaPO_4$ in 10% $(NH_4)_2SO_4$ at pH 7.
16. Load on to a column of phenyl sepharose (Cl 4B).
17. Wash with 15% $(NH_4)_2SO_4$.
18. Elute with 8% $(NH_4)_2SO_4$.
19. Monitor eluate, e.g. with HPLC analytical chromatography, and pool active samples.
20. Dialyse and freeze dry.
21. Dissolve to 25 mg per ml protein in 0.2M $(NH_4)HCO_3$ at pH 8.3 and load on column of Sephadex G-50.
22. Elute with 0.2M $(NH_4)HCO_3$ at pH 8.3 and monitor eluate, e.g. with HPLC. Approximately 1 g of pure freeze dried tPA inhibitor is obtained per kilogram seed.

The procedure substantially as described above was applied to samples of seeds of a number of Erythrina species and he tPA-inhibitor activity of each final product was determined as in Example 2. Typical results are given in Table 1 below.

TABLE 1

| Species | IU/mg |
|---|---|
| E. acanthrocarpa | 78 000 |
| E. caffra | 68 500 |
| E. corallodendron | 63 300 |
| E. lysistemon | 94 300 |
| E. seyheri | 71 400 |

Example 5

HPLC Analyses of tPA inhibitor purified from seeds of E. caffra.

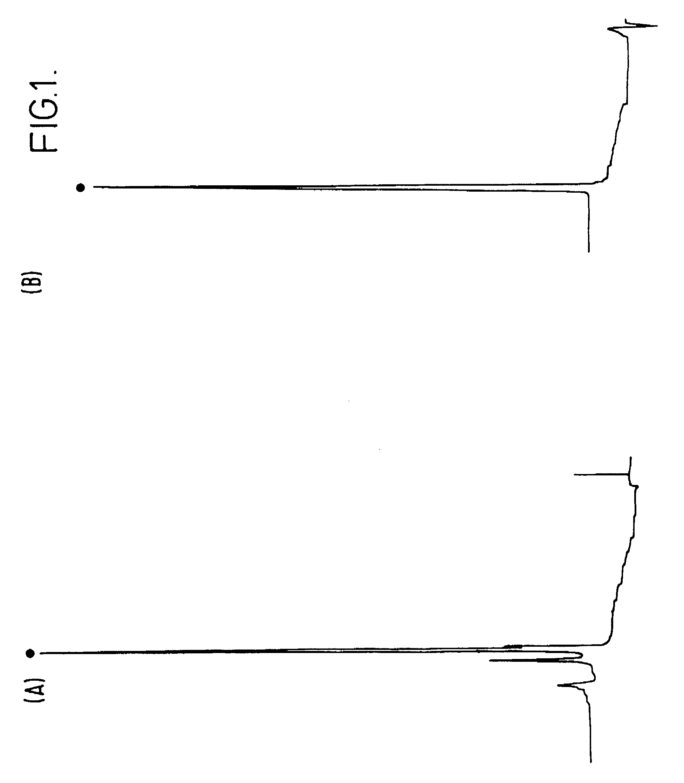

tPA inhibitor was purified from E.caffra seeds by the method of Joubert et al. (Hoppe-Seyler's Z. Physiol. Chem. 362, 531-534). The DE-3 peak of the DEAE-Sepharose column was dialyzed and lyophilized to yield a product with 52,000 IU/mg (Sample 1).
tPA inhibitor was also purified from E.caffra seeds by the method of Example 4. The final product was found to have an activity of 80,000 IU/mg (Sample 2).
Both samples of purified tPA inhibitor were analyzed by HPLC using a Waters HPLC chromatography system fitted with a Cl8 column. A linear gradient of 0.05% trifluoroacetic acid in water to 0.05% trifluoroacetic acid in 70% acetonitrile was pumped through the column at 1ml/min and eluted protein was detected by U.V. absorbance at 205nm.
The HPLC profiles obtained using approximately equal amounts of samples 1 and 2 are shown in Figures 1a and 1b respectively.
Figure 1a, but not Figure 1b, shows contaminating peaks. From Figure 1a, it can be deduced that approximately 20% of the protein present in Sample 1 was not tPA inhibitor.

Example 6

Analyses of tPA inhibitor purified from seeds of E.caffra and E.latissima by SDS-PAGE

(i) tPA inhibitor preparations
Three tPA inhibitor preparations were analyzed:

1. tPA inhibitor purified from E.caffra seeds by the method of Joubert et al. (Hoppe-Seyler's Z. Physiol Chem. (1981) 362, 531-538).
2. tPA inhibitor purified from E.latissima seeds by the method of Joubert et al (supra).
3. tPA inhibitor purified from E.caffra seeds by the method of Example 4.

(ii) SDS-PAGE
Electrophoresis was carried out on 15% polyacrylamide get slabs in the preence of 0.1% SDS. Bands were identified by Coomassie Brilliant Blue (CBB) staining , silver staining or the "Western Blot" technique employing a monoclonal antibody to the tPA inhibitor.
(iii) Results
With preparation 2 derived from E.latissima seeds only a single band was observed when staining was carried out with CBB, but two bands were observed by the "Western Blot" technique.
0.01 mg of preparation 3 migrated with a relative molecular mass (Mr) of approximately 20,000 and appeared on gels stained with CBB as a single homogeneous band. Analysis of preparation 1 in the same way showed a predominant component with a Mr of 20,000 and two minor contaminating proteins - the first with an Mr of approximately 22,000 and the second with an Mr of approximately 23,000.
When identical gels obtained following SDS-PAGE of 0.01 mg of preparations 1 and 3 were transferred on to nitrocellulose sheets for analysis by the "Western Blot" technique, the monoclonal antibody gave a strong signal with a 20,000 Mr protein in the case of both preparations. In the case of preparation 1, the antibody also bound, however, to an epitope on a trace 18,000 Mr contaminant. The sensitivity of the immunochemical technique was such that the protein was clearly visible despite the fact that it was only barely perceptible in a silver-stained get and not visible at all in the CBB-stained get. The 18,000 Mr band was not seen in the Western Blot obtained using preparation 3.

Example 7

Protein Fractions Present In the Seeds of Various Species of Erythrina

Table II below gives the results of molecular weight determinations of protein fractions isolated from seeds of various species of Erythrina by the method of Joubert et al. (Hoppe Seyler's Z.Physiol. Chem (1981) 362, 531-538)

TABLE II

| Fraction | E. latissima | E. caffra | E. lysistemon | E. corallo-dendron | E. Crista-galli | E. acanthro-carpa | E. humeana | E. seyheri |
|---|---|---|---|---|---|---|---|---|
| DE-1 | 22400 | 18604 | 19100 | 19000 | 19000 | 17400 | ? | 17800 |
| DE-2 | 22000 | 18600 | 18300 | 20000 | - | 18100 | ? | 17700 |
| DE-3 | 22000 | 18800 | 18800 | 20000 | 20000 | ? | 18200 | 16400 |
| DE-4 | - | 18800 | 18700 | - | 19000 | ? | | 18100 |
| DE-5 | - | - | - | 19000 | ? | ' | | 16900 |
| DE-6 | - | - | - | 20000 | ? | - | | - |
| DE-7 | - | - | - | 20000 | ? | - | | - |
| DE-8 | - | - | - | 19000 | 20000 | - | | - |

Molecular weight determinations were done by SDS gel electrophoresis as follows:-

(i) In the case of E.latissima seeds, by the method of Shapiro et al. Biochem. Biophys. Res. Comm. (1967) 28, 815-830.

13

(ii) In the case of E. caffra, E. lysistemon and E.humeana seeds by the method of Weber and Osborn. J. Biol. Chem. (1969)144, 4406.

(iii) In the case of E.lysistemon, E.cristagalli, and E.acanthrocarpa seeds by the method of Ornstein and Davis. (1962) "Disc electrophoresis", reprinted by Distillation Products Industries, Rochester, N.Y.

(iv) In the case of E.seyheri seeds by the method of Andrews. Methods Biochem. Anal. (1970), 18, 1.

The fractions which showed strong tPA inhibition activity are underlined. These results further illustrate that purification of tPA inhibitor from seeds of an Erythrina species free of contaminating proteins depends upon complete separation of the tPA inhibitor from other proteins of similar molecular weight. As indicated by the results of Examples 5 and 6, this is achieved by the method of Example 4, but not by the method of Joubert et al. (supra).

Example 8

Effect of Plant Proteases Present In the Seeds of Erythrina Species on tPA

Erythrina seed extracts were found to contain proteolytic activity that is measurable in the standard fluorometric assay, of Zimmerman et al (supra) by quantitating the catalysed release of amino-methyl coumarin (AMC) from the synthetic substrate Cbz-Gly-Gly-Arg-AMC.

Using the same fluorometric assay, tPA inhibitor preparations obtained from seeds of E. caffra by the method of Joubert et al. (supra) were also found to contain proteolytic activity (approx 1.4FU/mg protein). No such amidolytic activity was measureable in a tPA inhibitor preparation derived from E.caffra seeds by the method of Example 4.

A sample containing 31.4 FU/ml of tPA was mixed with an equal volume of a solution of protease derived from E. caffra seeds that contained 20.6 FU/ml and that had been established, by electrophoretic and immunochemical analysis, to be free of tPA inhibitor. The resulting mixture contained, as expected, 26.0 FU/ml. This solution was incubated at room temperature for 2 hrs and samples were taken for fluorometric assay at the time points indicated in Table III below.

TABLE III

| Time | Mixture | Erythrina Protease | Residual tPA | tPA |
|------|---------|--------------------|--------------|-----|
|      | FU/ml   | FU/ml              | FU/ml        | %   |
| 0    | 26.0    | 10.3               | 15.7         | 100 |
| 15   | 16.7    |                    | 6.4          | 40.8|
| 30   | 14.1    |                    | 3.8          | 24.2|
| 45   | 13.7    |                    | 3.4          | 21.7|
| 90   | 12.5    |                    | 2.2          | 14.0|
| 120  | 10.9    | 10.6               | 0.5          | 3.8 |

It is clear from this experiment that the Erythrina protease is capable of destroying the catalytic activity of tPA. This effect was seen in a period of exposure of the tPA to the Erythrina protease that might reasonably be expected to obtain under preparative circumstances where solutions containing tPA would be pumped on to an affinity column over the course of several hours.

Example 9

Cytotoxicity tests

UCT-BR-1 cells (derived originally from an osseous metastatic deposit from a primary carcinoma of the breast) were seeded in a 96-well microtitre tissue-culture plate at a density of $1.8 \times 10^4$ cells/well in 0.1 ml of RPMI-1640 medium supplemented with 10% foetal bovine serum and antibiotics. After 24 hrs of incubation at 37°c in a humid atmosphere of 95% air-5%$CO_2$ (by which time cells were adherent and subconfluent), tPA inhibitor purifed from seeds of an Erythina species by the method of Example 4 and dissolved in sterile RPMI-10 was added at different test concentrations. After a further 48 hrs of incubation, each well was pulsed for 18 hours with $^3$H-thymidine at a concentration of 0.1μg and 0.2 μCi/well.

Cells were then harvested and radioactivity incorporated into cellular DNA was measured. Cytotoxicity was measured as the inhibition of $^3$H-thymidine incorporation expressed as a percentage of the tPA inhibitor free-control.

Even when tPA inhibitor was present at a concentration as high as 1.0mg/ml, no measureable effect on cellular proliferation or DNA synthesis was found.

Essentially similar results were obtained using cells derived from normal human breast tissue.

The lack of cytotoxicity of the tPA inhibitor was confirmed by experiments in which this protein was administered to mice by subcutaneous intramuscular injection or intraperitoneal injection.

Example 10

Amino acid Sequence Analysis of The Trypsin Active Site Regions of tPA Inhibitors Derived From A Number of Erythrina Species.

tPA inhibitors purified from a variety of seeds of Erythrina species were subjected to limited hydrolysis at pH 3 by trypsin. Digestion was carried out for 7 days at 20 °C. The amino acid sequence around the trypsin reactive site of each tPA inhibitor was determined using fragments concentrated by SDS get electrophoresis in the presence of a reducing agent and the standard EDMAN procedures. Amino acid residues 63 to 74 are set out in Table IV below. The trypsin reactive site in each case is $Arg_{63}$-$Ser_{64}$.

## TABLE IV

| | 63 | 64 | 65 | 66 | 67 | 68 | 69 | 70 | 71 | 72 | 73 | 74 |
|---|---|---|---|---|---|---|---|---|---|---|---|---|
| E. caffra | -Arg*- | Ser- | Ala- | Phe- | Ile- | Pro- | Asp- | Asp- | Asp- | Lys- | Val- | Arg |
| E. decora | -Arg*- | Ser- | Thr- | Phe- | Ile- | Pro- | Asp- | Asp- | Asp- | Glu- | Val- | Arg |
| E. latissima | -Arg*- | Ser- | Thr- | Phe- | Ile- | Pro- | Asp- | Asp- | Asp- | Glu- | Val- | Arg |
| E. lysistemon | -Arg*- | Ser- | Ala- | Phe- | Ile- | Pro- | Asp- | Asp- | Asp- | Lys- | Val- | Arg |
| E. seyheri | -Arg*- | Ser- | Ala- | Phe- | Ile- | Pro- | Asp- | Asp- | Asp- | Lys- | Val- | Arg |
| E. acanthrocarpa | -Arg*- | Ser- | Thr- | Phe- | Ile- | Pro- | Asp- | Asp- | Asp- | Lys- | Val- | Arg |

From a comparison of the above sequences the following conclusions can be drawn:

(1) There is greater than 90% homology between the above fragments.
(2) Some departure from the amino acid sequences of E. latissima and E. caffra tPA inhibitors can be tolerated without lose of ability to inhibit tPA.
(3) Some variations are possible without elimination of tPA inhibiting activity, although the degree of activity may increase or decrease depending on such variations.

Example 11

Comparison of Amino Acid Compositions of tPA Inhibitors From Various Erythrina Species.

The amino acid compositious were determined of seven tPA inhibitor preparations from seeds of different Erythrina species. The results are set out in Table V below:

TABLE V

| Amino Acid | E. acan* | E. caffra* | E. coral ∗ | E. lat* | E. lys* | E. sey* | E. dec* |
|---|---|---|---|---|---|---|---|
| Asp | 18 | 16 | 15 | 17 | 16 | 17 | 15 |
| Thr | 9 | 8 | 9 | 9 | 8 | 8 | 9 |
| Ser | 14 | 13 | 14 | 13 | 14 | 14 | 12 |
| Glu | 23 | 25 | 21 | 25 | 26 | 24 | 23 |
| Pro | 11 | 10 | 14 | 10 | 10 | 10 | 13 |
| Gly | 14 | 14 | 15 | 14 | 14 | 14 | 14 |
| Ala | 6 | 7 | 8 | 6 | 6 | 7 | 7 |
| Cys | 4 | 4 | 4 | 4 | 4 | 4 | 4 |
| Val | 15 | 16 | 15 | 16 | 15 | 15 | 15 |
| Met | 0 | 0 | 1 | 0 | 0 | 0 | 1 |
| Ile | 6 | 6 | 6 | 6 | 7 | 7 | 6 |
| Leu | 16 | 16 | 16 | 16 | 16 | 16 | 16 |
| Tyr | 7 | 8 | 8 | 7 | 8 | 7 | 7 |
| Phe | 6 | 5 | 5 | 5 | 4 | 5 | 5 |
| Lys | 12 | 12 | 10 | 12 | 12 | 13 | 13 |
| His | 2 | 2 | 2 | 2 | 2 | 2 | 3 |
| Arg | 7 | 7 | 7 | 7 | 8 | 7 | 5 |
| Trp | 2 | 3 | 2 | 3 | 2 | 2 | 3 |
| TOTAL | 172 | 172 | 172 | 172 | 172 | 172 | 172 |

The above table permits some degree of correlation between the amino acid sequences of the inhibitors.
*Abbreviations
E. acan - E. acanthrocarpa, E.coral - E.corallodendron
E.lat - E.latissima, E.lys - E.lysistemon
E.sey - E.seyheri, E.dec. - E.decora

Example 12

Investigation of the importance of the disulphide bridges in a tPA inhibitor derived from seeds of an Erythrina species for inhibiting activity.

All Kunitz-type protease inhibitors derived from seeds of Erythrina species contain two disulphide bonds.

Limited hydrolysis of such an inhibitor using trypsin at pH 3 (Birk et al (1967) Biochem. Biophys. Acta 147, 402-404) without reduction was found to yield two disulphide-linked fragments which retain inhibitor activity towards tPA.

When, however, limited hydrolysis was followed by reduction of the two disulphide bridges and S-carboxymethylation with iodoacetate, tPA inhibition activity was lost.

Reduction of the disulphide bridges of the intact tPA inhibitor followed by S-carboxymethylation also resulted in loss of tPA inhibition activity.

These studies support the view that the two disulphide bridges are of importance for tPA inhibition activity.

Example 13

Isolation of tPA and pro-tPA from a human melanoma cell culture medium by affinity chromatography.

(i) Preparation of immobilised tPA inhibitor for use as the affinity reagent.

tPA inhibitor was isolated from Erythrina caffra seeds as described in Example 4. To prepare immobilised tPA inhibitor suitable for use as an affinity reagent, 200mg of the purified inhibitor was coupled to 40ml of swollen CNBr - activated Sepharose 4B (Pharmacia) according to the manufacturer's instructions. Coupling was done at room temperature and was monitored by $A_{280}$ measurements on the supernatant and by SDS-PAGE. Within 2½ hours, no protein was detectable in soluion by spectrum and his was confirmed by SDS-PAGE.

Immobilised tPA inhibitor was prepared for immediate use by equilibration in buffer (0.01M sodium phosphate, pH 7.4, containing 0.4M sodium chloride 0.1% Triton X-100, and 0.02% sodium azide as stabilizer).

The prepared affinity reagent was then packed into a column (2.3 x 9.6cm) ready for use.

As an alternative procedure, the inhibitor was also immobilised on other matrices which are more mechanically stable then Sepharose 4B, such as Fractogel or cross-linked agarose, to facilitate batch processing on large scale production as a possible alternative to the column method.

(ii) Cell culture and preparation of tPA/pro-tPA containing conditioned medium.

The human melanoma cell line RPMI 7272 (Bowes melanoma), established by Dr. George Moore of Denver, Colardo, provided a source of tPA. A subculture of this line was provided by Dr. E. Reich of the Rockefeller University, New York. Cells were maintained in 75 cm$^2$ Falcon flasks at 37°C in a humid atmosphere of 95% air and 5%CO$_2$. Confluent adherent monolayer cultures contained approximately 1 x 10$^7$ cells. These were covered with 20ml of RPMI 1640 medium supplemented with 10% heat-inactivated foetal bovine serum, 300 units/ml of penicillin and 200 μg/ml of streptomycin. Serum-free harvest fluids were collected by washing the monolayers several times with pre-warmed (37 °C) RPMI 1640 and covering the cells with 20ml of the same medium. After 24 hrs the tPA/pro-tPA containing conditioned medium was collected and replaced with fresh serum-free medium. Several (usually 3-4) sequential 24 hr serum-free harvest fluids could be collected before the cultures required rejuvenation by re-exposure to serumcontaining medium. Aprotinin (Sigma) was added to media at a concentration of 20 KIU/ml to prevent conversion of one-chain pro-tPA to two-chain tPA.

For storage, the harvest fluids were centrifuged to remove cellular debris, and made 0.1% with respect to Triton X-100. Harvest fluids treated in this way could be kept at -20°C for at least 6 months without apparent loss of enzyme activity.

(iii) Affinity chromatography

Batches of stored melanoma-cell harvest fluid prepared as in (ii) above were thawed and adjusted to pH 7.4 with 0.1M NaOH and made 0.4M with respect to NaCl and 0.1% w.r.t. Triton X-100. Sodium azide was added to a final concentration of 0.02%. Each batch, varying in volume from 1 to 8 litres, was then filtered through Whatman 541 paper and pumped, at 90 ml/hr and 4°C, through 40 ml column of tPA inhibitor-agarose prepared as in (i) above. In all cases, the column effectively removed all enzyme activity from the harvest fluid. Subsequent washing with 5 column volumes of equilibration buffer failed to displace the enzyme. Elution of the tPA/pro tPA was achieved with a solution of 1.6M KSCN in equilibration buffer.

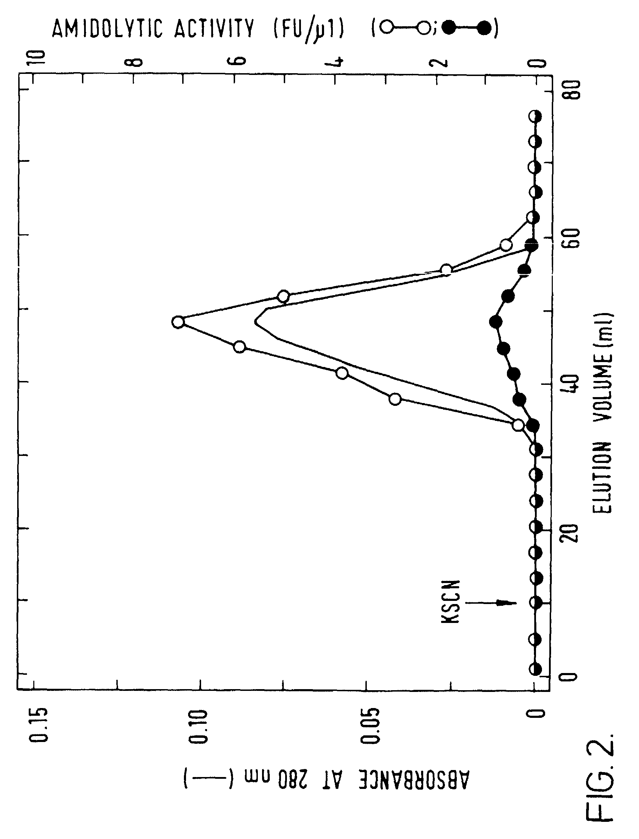

The results of a typical chromatographic run are presented in Fig. 2. In this experiment 6 litres of harvest fluid that had been collected in the presence of aprotinin were pumped through the column. The non-absorbed fraction contained no measurable tPA activity but a large number of other proteins that were visible on SDS-PAGE. The adsorbed tPA/pro-tPA was eluted with 1.6M KSCN as a single peak of amidolytic activity that corresponded to a single similarly-shaped peak of 280 nm absorption. Amidolytic activity in this peak (as determined by the method of Zimmerman et al. Proc. Nat. Acad Sci. U.S.A. (1978) 75, 750) was increased approximately 8-fold by treatment with plasmin, indicating that this fraction contained mainly one-chain pro-tPA.

Analyses of the enzyme-containing peak showed that the column was highly selective as an affinity reagent for tPA/pro-tPA and that most of the enzyme was, indeed, in the one-chain form. Electrophoresis of the purified enzyme under non-reducing conditions showed two prominent, closely-spaced protein bands with apparent molecular weights of 73 000 and 70 000 daltons. A third, less strong band at 68 000 daltons was also visible. In some runs traces of a 115 000 Mr species were observed. All of these corresponded to bands of fibrinolytic activity that were specifically inhibited by antibody raised to the 70 000 dalton tPA component. No proteins other than those of the tPA family were seen in silver-stained gels of the purified enzyme sample.

When electrophoresed under reducing conditions, the 72 000 dalton doublet remained and only traces of two bands with aparent molecular weights of 38 000 and 35 000 became visible. Incubation of the eluted enzyme with plasmin converted all of the protein to the 38 000 and 35 000 dalton forms.

We present a summary of the results of six purification runs. On average, melanoma cell conditioned media contained approximately 90 mg of protein/litre of which 0.3 mg/litre was adsorbed to the column and eluted with KSCN. The total tPA content of the harvest fluid was approximately 15 000 FU/litre. The KSCN eluate contained an average of 96% of the amidolytic activity that had been purified 270-fold.

Satisfactory elution of tPA from the tPA inhibitor affinity reagent at the end of the purification process can also be achieved with other solutions such as an acid urea solution (2M urea, 0.2M NH$_4$ OAc, pH 4).

Example 14

Purification of tPA and pro-tPA obtained by recombinant DNA technology using immobilised tPA-inhibitor.

tPA/pro-tPA containing solutions were obtained by the application of recombinant DNA technology as previously disclosed in European Patent Application 0093619 (4/5/1983).

A tPA-encoding gene was inserted into an expression vector at a suitable site for expression and the resulting vector was used to transform host cells. The transformed host cell line in which the tPA-encoding gene was expressed was maintained on a solid support in liquid medium or by growth in suspension containing supporting nutrients. Nutrients were added mainly by addition of serum to the medium. Anti-plasmins e.g. aprotinin, were also added prior to harvesting to prevent undesirable conversion of single chain tPA (pro-tPA) to the double chain form.

In this method, harvest fluid containing tPA/pro-tPA contains also undesirable serum proteins which should be removed in order to obtain medical grade tPA/pro-tPA suitable for treatment of human patients.

The purification of tPA/pro-tPA from serum enriched medium was achieved in the following way:-

tPA/pro-tPA containing solutions of neutral pH or slightly higher were loaded onto a 1.5 X 7.5cm column of immobilised tPA inhibitor prepared as in Example 6 (i) at approx. 30ml per hour at 4°C. The adsorbed tPA/pro-tPA was eluted with 1.6M KSCN. The recovery of tPA was 90% as measured by $A_{280}$ and tPA activity. The purity of the eluted tPA was 85% as measured by specific activity and SDS-PAGE.

Column capacity was found to be 2-5 mg tPA per ml. The column capacity of an identical column packed with immobilised tPA inhibitor prepared by the method of Joubert et al. (Joubert et al. (1981) Hoppe-Seyler's. Z. Physiol 362, 531-538) was found to be much lower-approx. 1mg tPA per ml of column.

In order to adequately purify tPA from serum containing media for medical use, further steps are required in addition to affinity purification using immobilised tPA-inhibitor as above. For example, the following protocol has been found to be suitable:

STEP 1 - affinity chromatography using immobilised tPA-inhibitor prepared as in Example 6(i) to reduce fluid volume and to obtain high recovery and high purity in one step.

STEP 2 - Carboxy-methyl-Sepharose (ion exchange purification) or lectins or HPLC.

STEP 3 - S-200 or S-300 (Pharmacia) (Sephacryl)

After a conventional polishing step, these three step processes product tPA of 98-100% purity.

## Claims

1. A process for the production of a protein reagent having the ability to bind to tPA and/or pro-tPA reversibly comprising a Kunitz-type proteinase inhibitor obtainable from seeds of an Erythrina species and having a molecular weight in the range 17-23,000 daltons, being non-cytotoxic, having the ability to inhibit tPA, pro-tPA and trypsin, but not urokinase or thrombin, having two disulphide bridges, having Val as the N-terminal amino acid residue and being free from proteases, wherein an aqueous solution containing said reagent together with contaminants is subjected to one or more fractionation steps including (a) chromatography using a hydrophobic get; (b) size-exclusion chromatography; and (c) ion-exchange chromatography; with selection of those fractions identified as containing said reagent and with exclusion of any fractions containing proteases.

2. A process for the production of a protein reagent having the ability to bind to tPA and/or pro-tPA reversibly comprising a Kunitz-type proteinase inhibitor having the following amino acid sequence:

$H_2$N-Val-Leu-Leu-Asp-Gly-Asn-Gly-Glu-Val-Val-  **10**

Gln-Asn-Gly-Gly-Thr-Tyr-Tyr-Leu-Leu-Pro-  **20**

Gln-Val-Trp-Ala-Gln-Gly-Gly-Gly-Val-Gln-  **30**

Leu-Ala-Lys-Thr-Gly-Glu-Glu-Thr-Cys-Pro-  **40**

Leu-Thr-Val-Val-Gln-Ser-Pro-Asn-Glu-Leu-  **50**

Ser-Asp-Gly-Lys-Pro-Ile-Arg-Ile-Glu-Ser-  **60**

Arg-Leu-Arg*-Ser-Ala-Phe-Ile-Pro-Asp-Asp-  **70**

Asp-Lys-Val-Arg-Ile-Gly-Phe-Ala-Tyr-Ala-  **80**

Pro-Lys-Cys-Ala-Pro-Ser-Pro-Trp-Trp-Thr-  **90**

Val-Val-Glu-Asp-Glu-Gln-Glu-Gly-Leu-Ser  **100**

Val-Lys-Leu-Ser-Glu-Asp-Glu-Ser-Thr-Gln-  **110**

Phe-Asp-Tyr-Pro-Phe-Lys-Phe-Glu-Gln-Val-  **120**

Ser-Asp-Gln-Leu-His-Ser-Tyr-Lys-Leu-Leu  **130**

Tyr-Cys-Glu-Gly-Lys-His-Glu-Lys-Cys-Ala  **140**

Ser-Ile-Gly-Ile-Asn-Arg-Asp-Gln-Lys-Gly-  **150**

Tyr-Arg-Arg-Leu-Val-Val-Thr-Glu-Asp-Tyr-  **160**

Pro-Leu-Thr-Val-Val-Leu-Lys-Lys-Asp-Glu-Ser-Ser-OH  **170**

and possessing two disulphide bridges in the following positions:

Cys 39 to Cys 83 and Cys 132 to Cys 139

or having a functionally analogous amino acid sequence having greater than 85% homology with the specified sequence in the region which includes positions 39 to 139, two disulphide bridges and Val in the N-terminal position, the protein reagent being non-cytotoxic, having the ability to inhibit tPA, pro-tPA and trypsin but not urokinase or thrombin and being free from proteases,

wherein an aqueous solution containing said reagent together with contaminants is subjected to one or more fractionation steps including (a) chromatography using a hydrophobic get; (b) size-exclusion chromatography; and (c) ion-exchange chromatography; with selection of those fractions identified as containing said reagent and with exclusion of any fractions containing proteases.

3. A process as claimed in claim 2 in which the said inhibitor has the following amino acid sequence:

                                                            10
       H₂N-Val-Leu-Leu-Asp-Gly-Asn-Gly-Glu-Val-Val-

                                                20
       Gln-Asn-Gly-Gly-Thr-Tyr-Tyr-Leu-Leu-Pro-

                                                30
       Gln-Val-Trp-Ala-Gln-Gly-Gly-Gly-Val-Gln-

                                                40
       Leu-Ala-Lys-Thr-Gly-Glu-Glu-Thr-Cys-Pro-

                                                50
       Leu-Thr-Val-Val-Gln-Ser-Pro-Asn-Glu-Leu-

                                                60
       Ser-Asp-Gly-Lys-Pro-Ile-Arg-Ile-Glu-Ser-

                                                70
       Arg-Leu-Arg*-Ser-Thr-Phe-Ile-Pro-Asp-Asp-

                                                80
       Asp-Glu-Val-Arg-Ile-Gly-Phe-Ala-Tyr-Ala-

                                                90
       Pro-Lys-Cys-Ala-Pro-Ser-Pro-Trp-Trp-Thr-

                                                100
       Val-Val-Glu-Asp-Glu-Gln-Glu-Gly-Leu-Ser

                                                110
       Val-Lys-Leu-Ser-Glu-Asp-Glu-Ser-Thr-Gln-

$$\overset{120}{\text{Phe-Asp-Tyr-Pro-Phe-Lys-Phe-Glu-Gln-Val-}} \quad \underset{}{=}$$

$$\overset{130}{\text{Ser-Asp-Lys-Leu-His-Ser-Tyr-Lys-Leu-Leu}}$$

$$\overset{140}{\text{Tyr-Cys-Glu-Gly-Lys-His-Glu-Lys-Cys-Ala}}$$

$$\overset{150}{\text{Ser-Ile-Gly-Ile-Asn-Arg-Asp-Gln-Lys-Gly-}}$$

$$\overset{160}{\text{Tyr-Arg-Arg-Leu-Val-Val-Thr-Glu-Asp-Asn-}}$$

$$\overset{170}{\text{Pro-Leu-Thr-Val-Val-Leu-Lys-Lys-Asp-Glu-Ser-Ser-OH}}$$

4. A process for the production of a protein reagent as claimed in any one of the preceding claims in which the said inhibitor constitutes at least 80% by weight of said protein reagent.

5. A process as claimed in claim 4 in which the said inhibitor constitutes at least 90% by weight of said protein reagent.

6. A process for the production of a protein reagent as claimed in any one of claims 1 to 3 in which the specific tPA and/or pro-tPA inhibiting activity of said protein reagent is at least 60,000 IU per milligram.

7. A process for the production of a protein reagent as defined in any one of the preceding claims which additionally comprises blocking the trypsin binding site of said reagent.

8. A process for the production of a protein reagent as defined in claim 1 which additionally comprises modifying said reagent by limited proteolysis with retention of the said two disulphide bridges.

9. A process for the production of a protein reagent as claimed in any one of the preceding claims which additionally comprises immobilising the said reagent on a carrier.

## Patentansprüche

1. Verfahren zur Herstellung eines Proteinreagens mit der Fähigkeit, reversibel an tPA und/oder Pro-tPA zu binden, mit einem Kunitz-Proteaseinhibitor, der aus Samen einer Erythrina-Art erhältlich ist, ein Molekulargewicht im Bereich 17000 - 23000 Dalton hat, nicht cytotoxisch ist, die Fähigkeit hat, tPA, Pro-tPA und Trypsin, nicht aber Urokinase oder Trombin zu inhibieren, der zwei Disulfidbrücken hat, der Val als N-Terminus-Aminosäurerest hat und frei von Proteasen ist, bei dem eine wäßrige Lösung, die das Reagens zusammen mit Verunreinigungen enthält, einem oder mehreren Fraktionierungsschritten unterworfen wird, einschließlich (A) Chromatographie unter Verwendung eines hydrophoben Gels; (B) Größenausschluß-Chromatographie; (C) Ionenaustausch-Chromatographie; mit Auswahl jener Fraktionen, die als das Reagens enthaltend identifiziert werden und Ausschluß aller proteasehaltigen Fraktionen.

2. Verfahren zur Herstellung eines Proteinreagens mit der Fähigkeit, tPA und/oder Pro-tPA reversibel zu binden, mit einem Kunitz-Proteaseinhibitor, der folgende Aminosäuresequenz hat:

H<sub>2</sub>N-Val-Leu-Leu-Asp-Gly-Asn-Gly-Glu-Val-Val-

Gln-Asn-Gly-Gly-Thr-Tyr-Tyr-Leu-Leu-Pro-

Gln-Val-Trp-Ala-Gln-Gly-Gly-Gly-Val-Gln-

Leu-Ala-Lys-Thr-Gly-Glu-Glu-Thr-Cys-Pro-

Leu-Thr-Val-Val-Gln-Ser-Pro-Asn-Glu-Leu-

Ser-Asp-Gly-Lys-Pro-Ile-Arg-Ile-Glu-Ser-

Arg-Leu-Arg*-Ser-Ala-Phe-Ile-Pro-Asp-Asp-

Asp-Lys-Val-Arg-Ile-Gly-Phe-Ala-Tyr-Ala-

Pro-Lys-Cys-Ala-Pro-Ser-Pro-Trp-Trp-Thr-

Val-Val-Glu-Asp-Glu-Gln-Glu-Gly-Leu-Ser-

Val-Lys-Leu-Ser-Glu-Asp-Glu-Ser-Thr-Gln-

Phe-Asp-Tyr-Pro-Phe-Lys-Phe-Glu-Gln-Val-

Ser-Asp-Gln-Leu-His-Ser-Tyr-Lys-Leu-Leu

Tyr-Cys-Glu-Gly-Lys-His-Glu-Lys-Cys-Ala

Ser-Ile-Gly-Ile-Asn-Arg-Asp-Gln-Lys-Gly-

Tyr-Arg-Arg-Leu-Val-Val-Thr-Glu-Asp-Tyr-

Pro-Leu-Thr-Val-Val-Leu-Lys-Lys-Asp-Glu-Ser-Ser-OH

und zwei Disulfidbrücken an den folgenden Positionen besitzt:

## Cys 39 nach Cys 83 und Cys 132 nach Cys 139,

oder der eine funktionsmäßig analoge Aminosäuresequenz besitzt, die mit der angegebenen Sequenz in dem Bereich, der die Positionen 39 bis 139 enthält, zu mehr als 85% homolog ist, zwei Disulfidbrücken hat und Val in der N-Terminusposition besitzt, wobei das Proteinreagens nicht cytotoxisch ist, die Fähigkeit hat, tPA, Pro-tPA und Trypsin, nicht aber Urokinase oder Trombin zu inhibieren und frei von Proteasen ist, bei dem eine wäßrige Lösung, die das Reagens zusammen mit Verunreinigungen enthält, einem oder mehreren Fraktionierungsschritten unterworfen wird, einschließlich (A) Chromatographie unter Verwendung eines hydrophoben Gels; (B) Größenausschluß-Chromatographie; (C) Ionenaustausch-Chromatographie; mit Auswahl jener Fraktionen, die als das Reagens enthaltend identifiziert werden und Ausschluß aller proteasehaltigen Fraktionen.

3. Verfahren nach Anspruch 2, bei dem der Inhibitor die folgende Aminosäuresequenz hat:

H₂N-Val-Leu-Leu-Asp-Gly-Asn-Gly-Glu-Val-Val-

Gln-Asn-Gly-Gly-Thr-Tyr-Tyr-Leu-Leu-Pro-

Gln-Val-Trp-Ala-Gln-Gly-Gly-Gly-Val-Gln-

Leu-Ala-Lys-Thr-Gly-Glu-Glu-Thr-Cys-Pro-

Leu-Thr-Val-Val-Gln-Ser-Pro-Asn-Glu-Leu-

Ser-Asp-Gly-Lys-Pro-Ile-Arg-Ile-Glu-Ser-

Arg-Leu-Arg*-Ser-Thr-Phe-Ile-Pro-Asp-Asp-

Asp-Glu-Val-Arg-Ile-Gly-Phe-Ala-Tyr-Ala-

Pro-Lys-Cys-Ala-Pro-Ser-Pro-Trp-Trp-Thr-

Val-Val-Glu-Asp-Glu-Gln-Glu-Gly-Leu-Ser-

Val-Lys-Leu-Ser-Glu-Asp-Glu-Ser-Thr-Gln-

Phe-Asp-Tyr-Pro-Phe-Lys-Phe-Glu-Gln-Val-

Ser-Asp-Lys-Leu-His-Ser-Tyr-Lys-Leu-Leu-

Tyr-Cys-Glu-Gly-Lys-His-Glu-Lys-Cys-Ala-

Ser-Ile-Gly-Ile-Asn-Arg-Asp-Gln-Lys-Gly-

Tyr-Arg-Arg-Leu-Val-Val-Thr-Glu-Asp-Asn-

Pro-Leu-Thr-Val-Val-Leu-Lys-Lys-Asp-Glu-Ser-Ser-OH

4. Verfahren zur Herstellung eines Proteinreagens nach einem der vorhergehenden Ansprüche, bei den der Inhibitor wenigstens 80 Gew.-% des Proteinreagens bildet.

5. Verfahren nach Anspruch 4, bei dem der Inhibitor wenigstens 90 Gew.-% des Proteinreagens bildet.

6. Verfahren zur Herstellung eines Proteinreagens nach einem der Ansprüche 1-3, bei dem die spezifische tPA- und/oder Pro-TPA-Inhibitionsaktivität des Proteinreagens wenigstens 60000 IU/mg beträgt.

7. Verfahren zur Herstellung eines Proteinreagens nach einem der vorhergehenden Ansprüche, welches zusätzlich eine Blockierung der Trypsin-Bindungsstelle des genannten Reagens beinhaltet.

8. Verfahren zur Herstellung eines Proteinreagens nach Anspruch 1, welches zusätzlich die Änderung dieses Reagens durch begrenzte Proteolyse unter Erhaltung der zwei Disulfidbrücken beinhaltet.

9. Verfahren zur Herstellung eines Proteinreagens nach einem der vorhergehenden Ansprüche, welches zusätzlich die Immobilisierung dieses Reagens auf einem Träger beinhaltet.

**Revendications**

1. Procédé de production d'un réactif protéique possédant la capacité de se lier de manière réversible au tPA et/ou au pro-tPA, comprenant un inhibiteur de la protéinase du type de Künitz, susceptible d'être obtenu à partir de graines d'une espèce d'érythrine et ayant une masse moléculaire comprise dans l'intervalle de 17 à 23 000 daltons, qui n'est pas toxique, a la capacité d'inhiber le tPA, le pro-tPA et la trypsine, mais non l'urokinase ou la thrombine, qui a deux ponts disulfure, qui présente Val comme résidu acide aminé N-terminal et est exempt de protéases,
   étant entendu qu'une solution aqueuse contenant ledit réactif avec des contaminants est soumise à une ou plusieurs étapes de fractionnement comprenant (a) chromatographie sur gel hydrophobe ; (b) chromatographie d'exclusion ; et (c) chromatographie par échange d'ions, avec sélection des fractions identifiées comme contenant ledit réactif et exclusion des fractions contenant des protéases.

2. Procédé de production d'un réacteur protéique ayant la capacité de se lier de façon réversible au tPA et/ou au pro-tPA, comprenant un inhibiteur de la protéinase du type de Künitz et ayant la séquence d'acides aminés suivante :

$$H_2N-Val-Leu-Leu-Asp-Gly-Asn-Gly-Glu-Val-\overset{10}{Val}-$$
$$Gln-Asn-Gly-Gly-Thr-Tyr-Tyr-Leu-Leu-\overset{20}{Pro}-$$
$$Gln-Val-Trp-Ala-Gln-Gly-Gly-Gly-Val-\overset{30}{Gln}-$$
$$Leu-Ala-Lys-Thr-Gly-Glu-Glu-Thr-Cys-\overset{40}{Pro}-$$
$$Leu-Thr-Val-Val-Gln-Ser-Pro-Asn-Glu-\overset{50}{Leu}-$$
$$Ser-Asp-Gly-Lys-Pro-Ile-Arg-Ile-Glu-\overset{60}{Ser}-$$
$$Arg-Leu-Arg^*-Ser-Ala-Phe-Ile-Pro-Asp-\overset{70}{Asp}-$$
$$Asp-Lys-Val-Arg-Ile-Gly-Phe-Ala-Tyr-\overset{80}{Ala}-$$
$$Pro-Lys-Cys-Ala-Pro-Ser-Pro-Trp-Trp-\overset{90}{Thr}-$$
$$Val-Val-Glu-Asp-Glu-Gln-Glu-Gly-Leu-\overset{100}{Ser}$$
$$Val-Lys-Leu-Ser-Glu-Asp-Glu-Ser-Thr-\overset{110}{Gln}-$$
$$Phe-Asp-Tyr-Pro-Phe-Lys-Phe-Glu-Gln-\overset{120}{Val}-$$

$$\begin{array}{c} 130 \\ \text{Ser-Asp-Gln-Leu-His-Ser-Tyr-Lys-Leu-Leu -} \\ 140 \\ \text{Tyr-Cys-Glu-Gly-Lys-His-Glu-Lys-Cys-Ala -} \\ 150 \\ \text{Ser-Ile-Gly-Ile-Asn-Arg-Asp-Gln-Lys-Gly -} \\ 160 \\ \text{Tyr-Arg-Arg-Leu-Val-Val-Thr-Glu-Asp-Tyr -} \\ 170 \\ \text{Pro-Leu-Thr-Val-Val-Leu-Lys-Lys-Asp-Glu - Ser-Ser-OH} \end{array}$$

et possédant deux ponts disulfure aux positions suivantes :

Cys 39 à Cys 83 et Cys 132 à Cys 139

ou ayant une séquence d'acides aminés fonctionnellement analogue ayant une homologie supérieure à 85 % avec la séquence spécifiée dans la région qui comprend les positions 39 à 139, deux ponts disulfure et Val à la position N-terminale, le réactif protéique étant non cytotoxique, ayant la capacité d'inhiber le tPA, le pro-tPA et la trypsine, mais non l'urokinase et la thrombine et étant exempt de protéases, étant entendu qu'une solution aqueuse contenant ledit réactif avec des contaminants est soumise à une ou plusieurs étapes de fractionnement comprenant (a) chromatographie sur gel hydrophobe ; (b) chromatographie d'exclusion ; et (c) chromatographie par échange d'ions, avec sélection des fractions identifiées comme contenant ledit réactif et exclusion des fractions contenant des protéases.

3.	Procédé selon la revendication 2, dans lequel ledit inhibiteur a la séquence d'acides aminés suivante :

$$\begin{array}{c} 10 \\ \text{H}_2\text{N-Val-Leu-Leu-Asp-Gly-Asn-Gly-Glu-Val-Val -} \\ 20 \\ \text{Gln-Asn-Gly-Gly-Thr-Tyr-Tyr-Leu-Leu-Pro -} \\ 30 \\ \text{Gln-Val-Trp-Ala-Gln-Gly-Gly-Gly-Val-Gln -} \\ 40 \\ \text{Leu-Ala-Lys-Thr-Gly-Glu-Glu-Thr-Cys-Pro -} \\ 50 \\ \text{Leu-Thr-Val-Val-Gln-Ser-Pro-Asn-Glu-Leu -} \\ 60 \\ \text{Ser-Asp-Gly-Lys-Pro-Ile-Arg-Ile-Glu-Ser -} \\ 70 \\ \text{Arg-Leu-Arg*-Ser-Thr-Phe-Ile-Pro-Asp-Asp -} \\ 80 \\ \text{Asp-Glu-Val-Arg-Ile-Gly-Phe-Ala-Tyr-Ala -} \end{array}$$

Pro-Lys-Cys-Ala-Pro-Ser-Pro-Trp-Trp- Thr -
                                    90

Val-Val-Glu-Asp-Glu-Gln-Glu-Gly-Leu- Ser
                                    100

Val-Lys-Leu-Ser-Glu-Asp-Glu-Ser-Thr- Gln -
                                    110

Phe-Asp-Tyr-Pro-Phe-Lys-Phe-Glu-Gln- Val -
                                    120

Ser-Asp-Lys-Leu-His-Ser-Tyr-Lys-Leu- Leu -
                                    130

Tyr-Cys-Glu-Gly-Lys-His-Glu-Lys-Cys- Ala -
                                    140

Ser-Ile-Gly-Ile-Asn-Arg-Asp-Gln-Lys- Gly -
                                    150

Tyr-Arg-Arg-Leu-Val-Val-Thr-Glu-Asp- Asn -
                                    160

Pro-Leu-Thr-Val-Val-Leu-Lys-Lys-Asp- Glu - Ser-Ser-OH
                                    170

4. Procédé de production d'un réactif protéique selon l'une quelconque des revendications précédentes, dans lequel ledit inhibiteur représente au moins 80 % en poids dudit réactif protéique.

5. Procédé selon la revendication 4, dans lequel ledit inhibiteur représente au moins 90 % en poids dudit réactif protéique.

6. Procédé de production d'un réactif protéique selon l'une quelconque des revendications 1 à 3, dans lequel l'activité spécifique inhibant le tPA et/ou le pro-tPA dudit réactif protéique est d'au moins 60 000 UI/mg.

7. Procédé de production d'un réactif protéique selon l'une quelconque des revendications précédentes, lequel comprend additionnellement le blocage du site de liaison à la trypsine dudit réactif.

8. Procédé de production d'un réactif protéique selon la revendication 1, lequel comprend additionnellement la modification dudit réactif par protéolyse limitée avec conservation desdits deux ponts disulfure.

9. Procédé de production d'un réactif protéique selon l'une quelconque des revendications précédentes, lequel comprend additionnellement l'immobilisation dudit réactif sur un support.

FIG.1.

(A)

(B)

FIG. 2.

EP 0 218 479 B2